# EUROPEAN PATENT SPECIFICATION

(11) **EP 2 453 845 B1**
(45) Date of publication and mention of the grant of the patent: **27.01.2016**
(21) Application number: 10737818.4
(22) Date of filing: 09.07.2010
(51) Int. Cl.: A61F 2/30, A61F 2/34, A61F 2/36, A61F 2/46, A61F 2/32

(54) **HIP PROSTHESIS**
HÜFTPROTHESE
PROTHÈSE DE HANCHE

(30) Priority: 17.07.2009 IT MC20090168
(43) Date of publication of application: 23.05.2012
(73) Proprietor: Melozzi, Alessandro, 64100 Teramo (TE) (IT)
(72) Inventor: Melozzi, Alessandro, 64100 Teramo (TE) (IT)
(74) Representative: Baldi, Claudio
(86) International application number: PCT/EP2010/059942
(87) International publication number: WO 2011/006852

(56) References cited:
- EP-A1- 1 380 274
- US-A- 4 846 840
- US-A1- 2005 137 710
- US-A1- 2009 043 397

## Description

The present patent application for industrial invention relates to a hip prosthesis conceived in such a way to save as much as possible the bone stock of the femur proximal end without giving the complications found in other prosthetic types, such as coating prosthesis.

As it is known, the anatomy of proximal femur has a spheroid surface, the femoral head, which articulates with a cavity of corresponding size situated in the pelvis, called cotyle or acetabulum. These two anatomic formations make up the coxofemoral joint.

The deterioration of articulation because of fractures or degenerative processes (coxarthrosis), when it cannot be treated with medications or physiotherapy, finds a solution in the implant of prosthesis.

Generally speaking, a typical hip prosthesis is made up of three components:
- a femoral component composed of a stem that is pressed inside the femoral channel, which is suitably modelled with progressive milling to generate a seat having the same shape as the stem; alternatively, a similar stem is cemented;
- a prosthetic head that is fixed onto the stem with a locking system technically known as "Morse taper";
- an acetabular cup complete with insert that articulates with the corresponding prosthetic head.

A detailed analysis of the components of a hip prosthesis shows that the stem is made of metal (normally titanium alloy), has a length of approximately 12-15 cm and a shape similar to the anatomic cast of the femoral channel or in any case a suitable shape to allow for being introduced and firmly fitted in the femur, adhering inside the channel on the internal walls of the bone corticals.

In order to introduce a stem of this type it is necessary to resect the femoral neck at the base and mill the inside of the femoral channel removing a large quantity of bone by using progressive rasps specific for each type of prosthesis. US 4846840 A discloses the features of the preamble of claim 1. US2004/0039449 discloses a prosthesis that does not save the neck. In fact the neck is totally removed. A long channel is obtained in the femur, in which a long prosthetic stem is inserted. Such prosthesis is not suitable for being mounted in the femur neck because is implanted in the femoral channel.

A small distal cup protrudes from the stem, being joined with the stem. The distal cup is shaped in section as an arch of circle with a subtended angle lower than 90°. The distal cup slides on the surface of a spherical segment, which is smaller than a hemisphere and totally contained inside a prosthetic head shaped as hemispheric segment. The centre of the distal cup is spaced from the centre of the hemispherical head. Filling elements are inserted in the peripheral part of the prosthetic head to form air spaces where the distal cup can slide.

Such a configuration is impaired by drawbacks caused by the presence of filling elements, which cause impingement and allow for limited regulation of the prosthesis. It must be noted that in such prosthesis it is impossible to regulate the offset, meaning the distance between the axis of the femur and the centre of rotation of the prosthetic head. Moreover, such a type of prosthesis is provided with very limited sliding surfaces, with consequent rapid wear.

In the "general economy" of the hip prosthesis implant the saving of bone stock is fundamental: the higher the bone quantity that is sacrificed during the implant, the more difficult the reimplant of a new prosthesis will be if, for whatever reason, the first implant prosthesis must be replaced (wear of materials, incorrect position, dislocation of components, etc.).

For this reason, design studies of new prosthesis are directed to find prosthesis systems that can reduce the loss of bone stock as much as possible.

In view of the above, in recent years the so-called "neck saving" prosthesis have been implanted in selected cases, in which the size and shape of stem are conceived in such a way that resection is performed not at the base of the femoral neck, but immediately under the head, implanting a stem that is able to anchor in the metaphisiary region without attacking the femoral channel. WO91/07932 discloses a prosthesis that saves the neck. A spherical head is fixed to the stem, resting on the corticals of the neck, with the risk of reabsorption. On the spherical head an acetabolar segment with hemispherical shape can slide. Also this type of configuration is impaired by regulation problems.

Moreover, it must be noted that, regardless of the size or shape, the femoral stem ends with a "Morse taper" on which the prosthetic head made of metal or ceramic is inserted, being articulated with the acetabolar component.

The latter is composed of a metal cup that is fixed to the pelvis either by means of pressure or screwing after suitably boring the natural cotyle. In the metal cup a ceramic or polyethylene insert is positioned, which articulates with the prosthetic head.

In case of fracture, especially in elder patients, the implant of the acetabolar component can be avoided, by inserting on the stem a head having the same size as the femoral head that has been fractured and removed, directly articulating the prosthetic head with the natural cotyle (endoprosthesis).

In addition to these prosthetic systems that, despite different profiles and different systems for anchoring to the bone, refer to a common "philosophy", the so-called "coating" prosthesis are available, in which no resection is performed, and the femoral head is reduced in volume with suitable boring bits, until it can be "coated" with a hemispherical metal dome that articulates with the acetabolar component.

The latter type of prosthesis has the great advantage of maintaining the entire femoral neck, although it is not free of complications, in first place the necrosis of the head and consequent mobilization of the prosthetic dome.

In any case, in addition to the skills of the orthopaedic surgeon, the correct operation and survival over time of the prosthesis depends on the quality of materials, the profile of the prosthesis and the surgical invasiveness on tissues.

Within such a technical situation, the purpose of the prosthesis of the present patent application is to obtain the highest saving of the bone stock, without the complications of the coating prosthesis, as well as reduce the friction coefficients of the couplings of prosthetic movement and allow for perfect integration between bone and stem prosthesis, thus almost completely saving the metaphisiary spongiosa.

The above has been achieved with a hip prosthesis according to the features of claim 1.

For purposes of clarity, the description of the invention continues with reference to the enclosed drawings, which are intended for purposes of illustration only and not in a limiting sense, wherein:
Fig. 1 is a diagrammatic view of a hip prosthesis;
Fig. 2 is a front view of a hip prosthesis according to an embodiment of the invention;
Fig. 3 is an axial sectional view of the prosthesis of Fig. 2;
Fig. 4 is an exploded axial sectional view of the prosthesis of Fig. 3;
Fig. 5 is a diagrammatic view that shows the prosthesis of Fig. 2 inserted in the femoral neck.

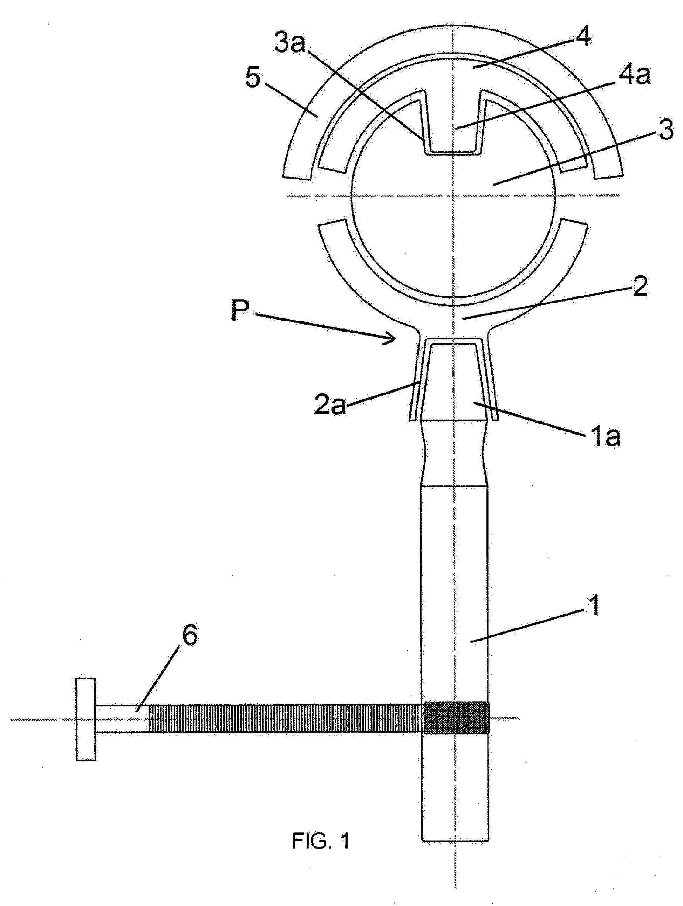

Referring to Fig. 1, the hip prosthesis is formed of:
- a basically cylindrical stem (1) ending on top with conical end (1 a),
- a prosthetic component (2) that is profiled as hemispherical cup and centrally provided in lower position with a conical cavity (2a) adapted to the fitted with said conical end (1 a) of the stem (1) with Morse taper system.
- a ball (3) adapted to be exactly fitted inside said prosthetic component (2) and centrally provided with a conical cavity (3a) on the side opposite said stem,
- a hemispherical prosthetic head (4) centrally provided with a conical appendix (4a) adapted to be fitted with the conical cavity (3a) of said ball (3) by means of "Morse taper" system, and
- a cup (5) adapted to be engaged in the acetabulum and exactly receive said hemispheric prosthetic head (4) with possibility of articulation.

As shown in Fig. 1, with respect to the intermediate ball (3), the hemispheric prosthetic head (4) is arranged in symmetrically opposite position with respect to said hemispheric prosthetic component (2). In particular, the centre of the hemispherical prosthetic component (2) coincides with the centre of the hemispherical prosthetic head (4).

Moreover, it must be noted that the diameter of the acetabolar cup (5) must be a few millimetres higher than the diameter of the hemispherical prosthetic component (2) to avoid impingement (i.e. dynamic interference) of the hemispherical prosthetic component (2) with respect to the border of the acetabolar cup (5).

At the end of the above description it is easier to understand the peculiarities of the prosthesis of the invention (P).

First, it must be noted that the empty fenestrated stem (1) favors maximum osteointegration, since it internally maintains (following to implant in the patient's femur) a cylinder of spongiosa that is nourished both by the base of the cylinder, which is left untouched, and by the neovascolarization favoured by the openings obtained on the stem (1).

The component that is defined as ball (3) is to be considered as an exclusive peculiarity of the prosthesis of the invention (P), since the presence of such a component is not found in the prior art.

The ball (3) can neither be considered as a femoral head nor assimilated to the shoulder inverse prosthesis.

Moreover, it must be noted that the sliding surface that guarantees the movement of the prosthesis of the invention (P) is formed by the coupling between the hemispherical prosthetic component (2) and the ball (3), and by the coupling between the prosthetic head (4) and the acetabolar cup (5), thus providing a sliding surface that is remarkably larger than any other prosthesis that is currently available on the market.

The importance of the sliding surface is clear if we consider that the wear of the material directly depends on the amount of sliding surface, being lower in case of higher load distribution surface.

The advantages of this prosthetic system can be summarized in three points:
- remarkable saving of femoral bone stock,
- reduction of wear coefficients between the mobile components of couplings for positioning of the intermediate ball that determines the doubling of the sliding surfaces between the components, with consequent reduction of prosthesis wear,
- maximum integration of the prosthetic stem because of the permanence inside the cylindrical cavity of the stem of a large quantity of spongiosa that, due to the presence of openings on the stem, will be progressively revascolarized and revitalized.

The results of such a prosthetic implant, after laboratory tests, and consequent marketing, can prove excellent in the clinical practice in the articular function and especially in the extension of prosthetic survival with evident reduction of problems for the patient and financial effort for the Health System.

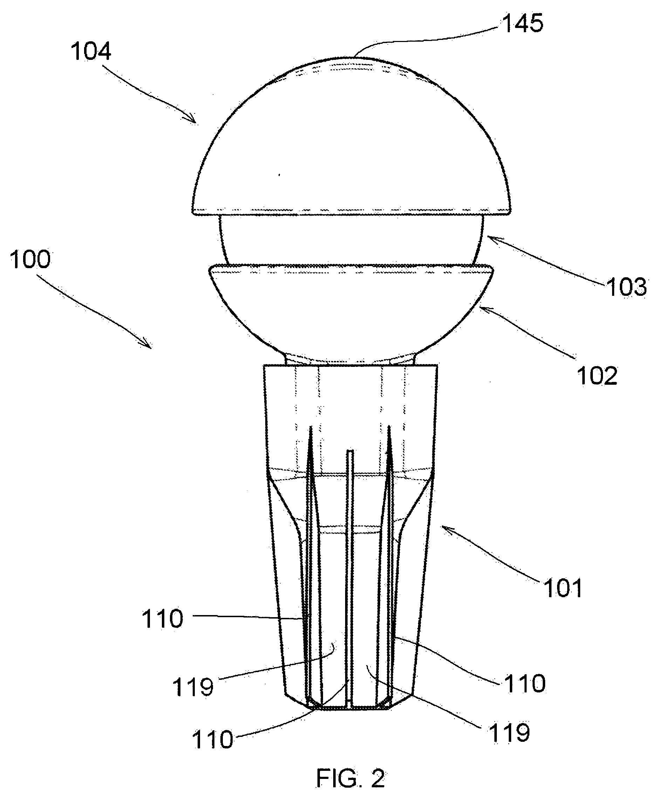

With reference to Figs. 2 - 5, a prosthesis (100) is disclosed according to an embodiment of the invention, in which the same elements or elements that correspond to the ones described above are indicated with the same reference numerals increased by 100, therefore omitting a detailed description.

The prosthesis (100) comprises a truncated-conical stem (101) provided with longitudinal wings (110) that protrude radially outwards to penetrate in the spongiosa of the femoral neck. Air spaces (119) are defined between the wings (111) for anchoring with the spongiosa.

An axial channel (111) is obtained inside the stem to allow for entrance of a guidewire. The stem (101) is provided with seats (112) open in the lower side to allow for entrance of spongiosa.

A truncated-conical seat (101 a) is obtained at the upper end of the stem, in which a truncated-conical shank (102a) that protrudes in lower position from the prosthetic component or distal cup (102) is inserted. Although not shown in the figures, the shank (102a) can be internally empty, shaped as traditional Morse taper to allow for coupling with the stem (101) of the invention and also with traditional stems.

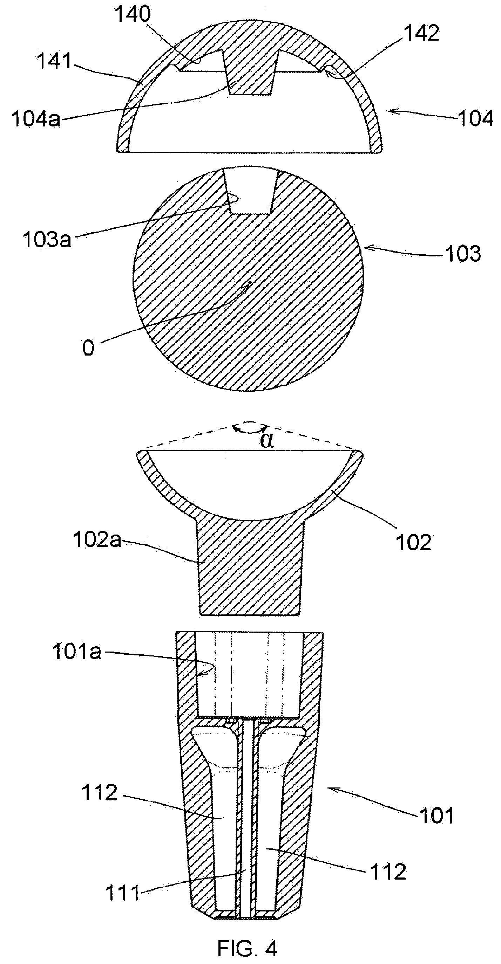

As shown in Fig. 4, the distal cup (102) seen as a cross-section is shaped as an arch of circle with central angle (α) slightly lower than 180°. Preferably the central angle (α) is higher than 160° to maximize the sliding surface and consequently minimize friction.

The ball (103) has a perfectly spherical external surface, that is to say a circular cross-section with centre (O). The ball (103) has a truncated-conical hole (103a) in which a truncated-conical shank (104a) that protrudes radially inwards from the central part of the prosthetic head (104) is fitted.

The prosthetic head (104) is shaped as a hemispherical segment, that is to say that seen in cross-section it is an arch of circle subtended by a central angle of approximately 180°.

The prosthetic head (104) has double sphericity, meaning that in the external surface of the prosthetic head a central portion (145) that protrudes outwards is provided, having a surface with basically ovoidal or ellipsoidal profile to reproduce as much as possible the anatomy of the femoral head, thus better adjusting in the acetabolar seat.

The segment (104) around the shank (104a) has a central portion (140) with higher thickness than a peripheral portion (141) in such a way to generate a stepped stop surface (142). Advantageously, the central portion (140) has double thickness with respect to the peripheral portion (141). For example, the central portion (140) has 4 mm thickness, whereas the peripheral portion (141) has 2 mm thickness. Moreover, the thickness of the proximal cup (102) is the same as the thickness of the peripheral portion (141) of the prosthetic head or in any case the same as the thickness of the step (142).

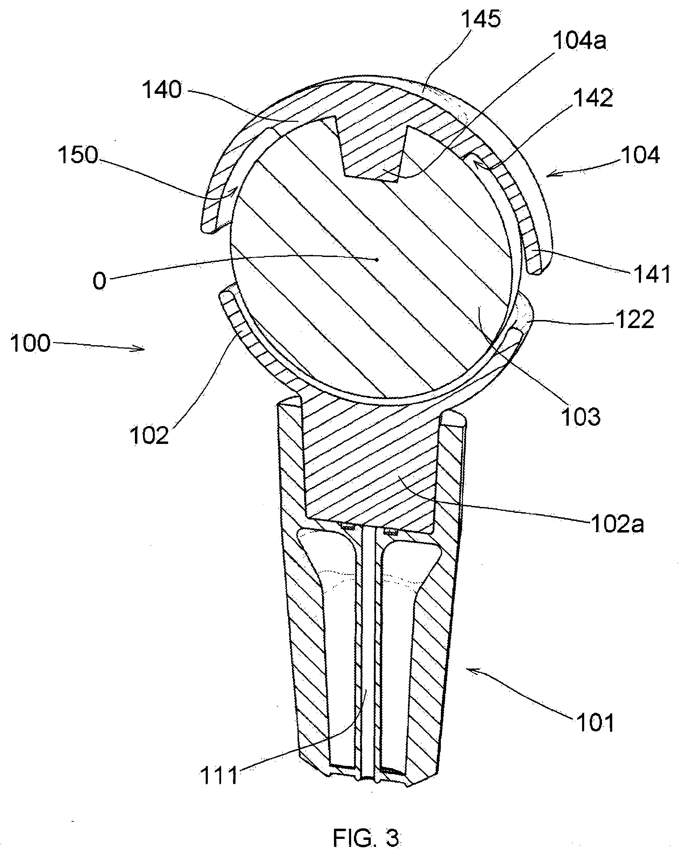

Referring to Fig. 3, when the prosthetic head (104) is mounted on the ball (103), the central portion (140) of the prosthetic head touches the external surface of the ball (103), thus generating an air space (150) between the external surface of the ball (103) and the internal surface of the peripheral portion (141) of the prosthetic head. Said air space (150) is adapted to receive the border (122) of the distal cup (102) that slides on the ball (103). When part of the distal cup (102) is in the air space (150) it is perfectly guided both by the ball (103) and the prosthetic head (104).

The relative rotation between prosthetic head (104) and distal cup (102) is blocked when the border (122) of the distal cup touches the stepped stop surface (142) of the prosthetic head. In such a condition, a blow or counterblow is generated in such a way that the prosthetic head (104) together with the ball (103) creates a movement.

Normally, the distal cup (102) is totally out of the prosthetic head (104). In any case, when the border (122) of the distal cup touches against the step (142) of the prosthetic head, most of the distal cup (102) is out of the prosthetic head.

Referring to Fig. 3, in normal conditions the axis of the shank (104a) coincides with the axis of the shank (102a) and channel (111). In case of a large travel of the stem (101) and distal cup (102) assembly with respect to the ball (103) and prosthetic head (104) assembly, the border (122) of the distal cup (102) touches against the step (142) of the prosthetic head (104). Accordingly, the prosthetic head (104) and ball (103) assembly receives a blow and self-centres, bringing back the axis of the shank (104a) in coincidence with the axis of the shank (102a), as shown in Fig. 3.

It must be noted that the centre of the radius of curvature of the distal cup (102), the centre (O) of the ball (103) and the centre of the radius of curvature of the prosthetic head (104) coincide. Said configuration allows for great freedom of rotation without impingement, perfect adjustment of the prosthesis, simply assembly and at the same time maximization of sliding surfaces.

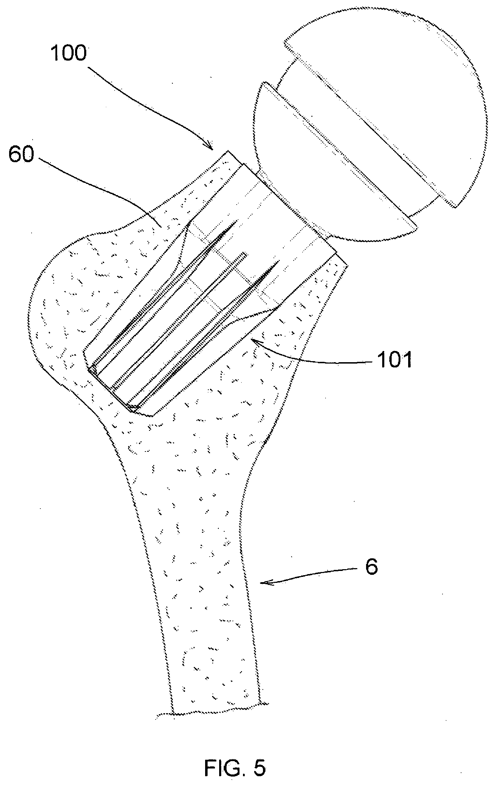

Fig. 5 shows the prosthesis (100) mounted in the neck (60) of the femur (6). As shown in the figure, said type of prosthesis allows for saving the neck (60) of the femur. The stem (101) must have a length lower than 65 mm and has been especially studied to anchor to the spongiosa of the femoral neck.

To understand the totally innovative precepts expressed by this new type of prosthesis brief reference must be made to surgical technique.

First of all, it must be said that after making pre-surgical planning, by means of suitable transparencies, it is necessary to choose the size of the stem (101), the prosthetic head (104) and consequently the ball (103) and the distal cup (102).

After performing a dislocation maneuver of the femoral epiphysis, the osteotomy resection level is determined. The osteotomy length is determined according to the system chosen during the pre-surgical planning.

The head osteotomy is performed exactly at the base, in such a way to have full visibility of the femoral neck in its projections.

Points of departure are traced on the osteotomy plane with a suitable instrument in order to perfectly identify the centre of the femoral neck. The surgeon enters in the femoral neck with a guidewire, passing beyond it and perforates the opposite cortical under the greater trochanter in such a way to obtain stability during the passage of the rasps.

The spongiosa contained in the femoral neck is perforated with a small-gauge mill, creating an invitation for the rasps that will prepare the seat for the final prosthesis.

After preparing the final seat of the prosthesis inside the neck of the femur with progressive rasps, the surgeon chooses the prosthesis (101) that corresponds to the measure of the last rasp used and takes the prosthesis directly from the sterile packaging. With a suitable impacter/extractor that uses the Morse taper of the male neck, introduced in the empty space of the stem, the surgeon turns the threaded handle that is blocked inside the internal cavity of the prosthesis. Then the final implant is performed.

The surgeon measures the acetabolar fundus with test heads to determine the final choice of the prosthetic head (104) and makes an additional check by measuring the femoral head removed with osteotomy with a gauge. After determining the size of the prosthetic head (104), the surgeon chooses the ball (103) and its distal cup (102) that will be connected with the final stem (101) by means of Morse taper.

The surgeon will reduce the prosthesis and will control the length of the limb and the stability of the prosthesis in order to check that the prosthesis does not dislocate.

## Claims

1. Hip prosthesis (P; 100) comprising:
- a prosthetic head (4; 104) shaped as a basically hemispherical cup **having a sliding outer surface** adapted to articulate with an acetabular cup (5) inserted in the acetabulum **and having at its inner surface a concavity,**
- a first prosthetic component (2; 102) having the shape of a basically hemispherical cup with concavity towards the concavity of said prosthetic head,
- a second prosthetic component (3; 103) connected to the prosthetic head and arranged inside said **first** prosthetic component,
**wherein**
- said second prosthetic component (3; 103) is a ball having a circular section with centre (O), and
- the centre of the radius of curvature of said first prosthetic component (2; 102) coincides with the centre of the radius of curvature of said prosthetic head (4; 104) that coincides with the centre (O) of said ball (3; 103),
**characterized in that**
**the hip prosthesis comprises a stem (1; 101) adapted to be inserted in the neck (60) of the femur (6) and first prosthetic component is connected to said stem (1; 101),**
**said ball (3; 103) comprises a** hole **(3a; 103a)** inside which a shank (4a; **104a) that protrudes radially inwards from said prosthetic head (4; 104) is coupled, and**
**said prosthetic head (104) comprises a central portion with higher thickness (140) arranged around said shank (104a) and a peripheral portion (141) with lower thickness, in such a way to generate a stop surface (142); said central portion with higher thickness (140) being disposed on the external surface of the ball (103) in such a way to generate an air space (150) between the external surface of the ball (103) and the internal surface of said peripheral portion with lower thickness (141), in such a way that the border (122) of said first component (102) can enter inside said air space (150) until it stops against said stop surface (142).**

2. Hip prosthesis (P; 100) as claimed in claim 1, **characterized in that** said first prosthetic component (2; 102) has a section shaped as arch of circle with a central angle (α) higher than 160 °.

3. Hip prosthesis (P; 100) as claimed in claim 1 or 2, **characterized in that** said first prosthetic component (2; 102) is at least partially out of said prosthetic head (4; 104).

4. Hip prosthesis (P; 100) as claimed in any one of the above claims, **characterized in that** said stem (1; 101) has length lower than 60 mm in order to be inserted in the neck (60) of the femur.

5. Hip prosthesis (100) as claimed in any one of the above claims, **characterized in that** said stem (101) has a truncated-conical shape with external longitudinal ribs (110) and an axial channel (111) open at the lower end for access of spongiosa.

6. Hip prosthesis (100) as claimed in claim 5, **characterized in that** said first component (102) is provided with a shank (102a) that protrudes radially outwards to couple inside a hole (101 a) in the upper part of said stem (101), said shank (102a) being internally empty in such a way to form a cavity in communication with said axial channel (111) of the stem for access of spongiosa.

7. Hip prosthesis (100) as claimed in any one of the above claims, **characterized in that** said prosthetic head (104) comprises a central portion (145) that protrudes outwards, having a surface with basically ovoidal or ellipsoidal profile.

## Patentansprüche

1. Hüftprothese (P; 100) umfassend:
- einen im Wesentlichen halbkugelförmigen Prothesenkopf (4; 104) mit einer äußeren Gleitfläche, die dazu geeignet ist, sich mit einer Gelenkpfanne (5) gelenkig zu verbinden, die im Pfannenknochen eingesetzt ist und eine Höhlung auf ihrer Innenfläche aufweist,
- ein erstes, im Wesentlichen halbkugelförmiges Prothesenteil (2; 102) mit einer zur Höhlung des Prothesenkopfes gerichteten Höhlung,
- ein zweites Prothesenteil (3; 103), das mit dem Prothesenkopf verbunden und im ersten Prothesenteil angeordnet ist,
wobei
- das zweite Prothesenteil (3; 103) eine Kugel mit einem kreisförmigen Querschnitt mit Mittelpunkt (O) ist; und
- der Mittelpunkt des Krümmungsradius des ersten Prothesenteils (2; 102) mit dem Mittelpunkt des Krümmungsradius des Prothesenkopfes (4; 104) zusammenfällt, der mit dem Mittelpunkt (O) der Kugel (3; 103) zusammenfällt,
**dadurch gekennzeichnet, dass**
die Hüftprothese einen Stiel (1; 101) umfasst, der dazu geeignet ist, in den Hals (60) des Oberschenkelknochens (6) eingesetzt zu werden und das erste Prothesenteil mit dem Stiel (1; 101) verbunden ist;
die Kugel (3; 103) eine Bohrung (3a; 103a) umfasst, in die ein Schaft (4a; 104a), der radial ins Innere des Prothesenkopfes (4; 104) auskragt, und
der Prothesenkopf (104) einen zentralen Anteil größerer Dicke (140) umfasst, der rund um den Schaft (104a) angeordnet ist, und einen peripheren Anteil (141) geringerer Dicke derart, dass eine Anschlagfläche (142) gebildet wird; wobei der zentrale Anteil größerer Dicke (140) auf der Außenfläche der Kugel (103) derart zur Anlage kommt, dass ein Zwischenraum (150) zwischen der Außenfläche der Kugel (103) und der Innenfläche des peripheren Anteils geringerer Dicke (141) gebildet wird, derart, dass der Rand (122) des ersten Prothesenteils (102) in den Zwischenraum (150) eindringen kann, bis er an der Anschlagfläche (142) in Anschlag geht.

2. Hüftprothese (P; 100) nach Anspruch 1, **dadurch gekennzeichnet, dass** das erste Prothesenteil (2; 102) einen kreisbogenförmigen Querschnitt aufweist, dessen Mittelpunktswinkel (α) größer als 160 ° ist.

3. Hüftprothese (P; 100) nach Anspruch 1 oder 2, **dadurch gekennzeichnet, dass** das erste Prothesenteil (2; 102) sich mindestens teilweise außerhalb des Prothesenkopfes (4; 104) befindet.

4. Hüftprothese (P; 100) nach einem beliebigen der vorstehenden Ansprüche, **dadurch gekennzeichnet, dass** der Stiel (1; 101) eine Länge von weniger als 60 mm aufweist, um in den Hals (60) des Oberschenkelknochens eingesetzt werden zu können.

5. Hüftprothese (100) nach einem beliebigen der vorstehenden Ansprüche, **dadurch gekennzeichnet, dass** der Stiel (101) die Form eines Kegelstumpfes mit äußeren Längsrippen (110) sowie einen axialen Kanal (111) aufweist, der unterseitig für das Einwachsen der Spongiosa offen ist.

6. Hüftprothese (100) nach Anspruch 5, **dadurch gekennzeichnet, dass** das erste Prothesenteil (102) einen Schaft (102a) aufweist, der radial nach außen auskragt, um sich in eine Bohrung (101 a) im oberen Teil des Stiels (101) einzukuppeln, wobei der Schaft (102a) inwendig hohl ist, derart, dass eine mit dem axialen Kanal (111) des Stiels kommunizierende Kavität für das Einwachsen der Spongiosa gebildet wird.

7. Hüftprothese (100) nach einem beliebigen der vorstehenden Ansprüche, **dadurch gekennzeichnet, dass** der Prothesenkopf (104) einen zentralen Anteil (145) umfasst, der nach außen auskragt und eine Oberfläche mit einem im Wesentlichen ovalen oder ellipsenförmigen Profil aufweist.

## Revendications

1. Prothèse de hanche (P; 100) comprenant :
- une tête de prothèse (4; 104) en forme de coupe essentiellement semi-sphérique ayant une surface extérieure de glissement adaptée pour s'articuler avec une cupule acétabulaire (5) logée dans le cotyle et présentant une concavité dans sa surface intérieure,
- un premier élément de prothèse (2; 102) ayant une forme de coupe essentiellement semi-sphérique dont la concavité est tournée vers la concavité de ladite tête prothétique,
- un deuxième élément de prothèse (3; 103) relié à la tête prothétique et disposé à l'intérieur dudit premier élément de prothèse,
où
- ledit deuxième élément de prothèse (3; 103) est une sphère ayant une section circulaire avec centre (O) ; et
- le centre du rayon de courbure dudit premier élément de prothèse (2; 102) coïncide avec le centre du rayon de courbure de ladite tête prothétique (4; 104) qui coïncide avec le centre (O) de ladite sphère (3; 103),
**caractérisée en ce que**
la prothèse de hanche comprend une tige (1; 101) apte à être logée dans le col (60) du fémur (6) et le premier élément de prothèse est relié à ladite tige (1; 101) ;
ladite sphère (3; 103) comprend un trou (3a; 103a) à l'intérieur duquel elle s'accouple à une queue (4a; 104a) qui fait saillie radialement vers l'intérieur à partir de ladite tête prothétique (4; 104), et
ladite tête prothétique (104) comprend une partie centrale de plus grande épaisseur (140) disposée autour de ladite queue (104a) et une partie périphérique (141) de plus faible épaisseur, de manière à engendrer un échelon de butée (142) ; ladite partie centrale de plus grande épaisseur (140) atteignant la surface extérieure de la sphère (103) de manière à générer un interstice (150) entre la surface extérieure de la sphère (103) et la surface intérieure de ladite partie périphérique de plus faible épaisseur (141), de sorte que le bord (122) dudit premier élément (102) peut entrer dans ledit interstice (150) jusqu'à arriver en butée contre ladite surface de butée (142).

2. Prothèse de hanche (P; 100) selon la revendication 1, **caractérisée en ce que** ledit premier élément de prothèse (2; 102) présente une section en arc de cercle sous-tendu par un angle au centre (α) supérieur à 160 °.

3. Prothèse de hanche (P; 100) selon la revendication 1 ou 2, **caractérisée en ce que** ledit premier élément de prothèse (2; 102) est disposé au moins partiellement à l'extérieur de ladite tête prothétique (4; 104).

4. Prothèse de hanche (P; 100) selon l'une quelconque des revendications précédentes, **caractérisée en ce que** ladite tige (1; 101) a une longueur inférieure à 60 mm, afin d'être insérée dans le col (60) du fémur.

5. Prothèse de hanche (100) selon l'une quelconque des revendications précédentes, **caractérisée en ce que** ladite tige (101) a une forme tronconique avec des nervures longitudinales externes (110) et un canal axial (111) ouvert vers le bas pour l'entrée de l'os spongieux.

6. Prothèse de hanche (100) selon la revendication 5, **caractérisée en ce que** ledit premier élément (102) comporte une queue (102a) qui fait saillie radialement vers l'extérieur pour s'accoupler dans un trou (101 a) dans la partie supérieure de ladite tige (101), ladite queue (102a) étant creuse à l'intérieur de manière à former une cavité communiquant avec ledit canal axial (111) de la tige pour l'entrée de l'os spongieux.

7. Prothèse de hanche (100) selon l'une quelconque des revendications précédentes, **caractérisée en ce que** la tête prothétique (104) comprend une partie centrale (145) qui fait saillie vers l'extérieur ayant une surface avec profil essentiellement ovoïde ou ellipsoïde.
